(19) Europäisches Patentamt European Patent Office Office européen des brevets

(11) **EP 4 300 068 B1**

(12) **EUROPEAN PATENT SPECIFICATION**

(45) Date of publication and mention of the grant of the patent:
**13.11.2024   Bulletin 2024/46**

(21) Application number: **23180879.1**

(22) Date of filing: **22.06.2023**

(51) International Patent Classification (IPC):
**G01N 1/08** *(2006.01)*         **B66F 11/04** *(2006.01)*
**G01N 1/12** *(2006.01)*         **G01N 33/02** *(2006.01)*
**G01N 33/00** *(2006.01)*

(52) Cooperative Patent Classification (CPC):
**G01N 1/08; G01N 1/12; G01N 33/0091;**
G01N 33/02; G01N 2001/1006

(54) **SAMPLING MECHANISM IN PARTICULAR FOR BULK MATERIALS**

PROBENENTNAHMEVORRICHTUNG, INSBESONDERE FÜR SCHÜTTGUT

DISPOSITIF DE PRÉLÈVEMENT D'ÉCHANTILLONS, EN PARTICULIER POUR DES PRODUITS EN VRAC

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:   **27.06.2022   PL 44156422**

(43) Date of publication of application:
**03.01.2024   Bulletin 2024/01**

(73) Proprietor: **CEREUS WENA ADAM I GRAZYNA WITKOWSCY SP. J.**
**87-100 Torun (PL)**

(72) Inventors:
 • **Wojtyra, Marek**
   **01-494 Warszawa (PL)**
 • **Mianowski, Krzysztof**
   **03-984 Warszawa (PL)**
 • **Kaminski, Grzegorz**
   **05-240 Tluszcz (PL)**
 • **Barczak, Tomasz**
   **03-721 Warszawa (PL)**
 • **Pekal, Marcin**
   **01-946 Warszawa (PL)**
 • **Surowiec, Marek**
   **05-840 Brwinow (PL)**
 • **Witkowski, Adam**
   **87-100 Torun (PL)**
 • **Witkowski, Marcin**
   **87-100 Torun (PL)**

(74) Representative: **Wroblewski, Marcin Jan**
**Fert, Jakubiak vel Wojtczak**
**Wróblewski rzecznicy patentowi**
**Sp. P. / ul. Gdanska 126/A103**
**90-520 Lodz (PL)**

(56) References cited:
**CN-U- 203 011 723       FR-A1- 2 542 086**
**US-A- 2 006 066         US-A- 3 789 671**

## Description

The subject of the invention is a sampling mechanism , in particular for bulk materials, intended for sampling devices for bulk materials, especially bulk materials of agricultural origin. The device is used, among others, in agriculture and in the grain and feed industry in general.

Bulk materials consist of particles of different densities and sizes, and it is impossible to perceive bulk materials present in a batch as a set of separate objects. To evaluate these materials, sampling devices are used to take a sample from the batch of material that is intended to be representative of the entire batch being analysed. In the activity related to the trading of bulk materials of agricultural origin, bulk material is transported in large quantities in tanks and containers by means of mass transport, such as trucks, railway wagons or barges. In such activity, it is necessary to comply with the established standards of quality control of agricultural products, because the quality of the product affects its intended use and price. Samples to determine the quality of the transported bulk material are collected using sampling devices that often operate in an automated manner.

In the solutions known in the state of the art, sampling devices for bulk materials have a vertical column permanently anchored in the ground, on which a driving module is mounted. The driving module has a base connected to the column and an arm connected thereto, at the free end of which a sampling probe is attached. The arm in many cases has a telescopic structure and is rotatably attached to the base, which allows the arm to move up and down. The task of the driving module is to insert the sampling probe into the material being collected. In the case of a telescopic arm, its length is set before driving the probe and remains constant during the driving process. Rotary mounting of the arm to the base results in guiding the probe in an arc while driving it into the bulk material. This causes the mechanical system to be loaded with forces resulting from the displacement of the probe immersed into the bulk material, not only in the direction along the axis of the probe, but also in the direction transverse to the axis of the probe. It also makes it difficult to operate the probe precisely. Furthermore, these difficulties are also caused by unpredictable, variable forces of interaction between the probe and the bulk material. In the case of operating close to the walls of containers with bulk material, the inability to precisely predict the trajectory of the movement of the probe may lead to a collision with elements of the environment and, consequently, to damage.

In the state of the art, there are also solutions with a gantry on which a sampling mechanism is seated. In such solutions, guides are used to allow the probe to move vertically while driving into the bulk material. However, these are solutions characterized by large dimensions, and thus also a high price.

The document ES 285485 U discloses a typical pneumatic device for sampling bulk products comprising a mast in the form of a stationary vertical column seated in the ground, on the upper part of which an arm in the form of a parallelogram is mounted. The arm has a kinematic structure of a parallelogram and consists of two longer connectors and two shorter connectors. At the end of the arm, a probe is attached to the shorter connector. The upward and downward movement of the arm is provided by a pneumatic cylinder mounted under the arm in the upper part of the vertical column.

While the patent document DE 3612410 A1 discloses a device for collecting soil samples comprising a structure with a pair of arms forming a parallelogram system connected to a support structure. The pair of arms is movably connected to vertical arms of the support structure. At the free end of the pair of arms, a probe in the form of a drill is mounted for collecting soil samples. The pair of arms forming a parallelogram is raised and lowered by hydraulic cylinders that are pivotally attached to the top of the vertical arm of the support structure frame.

Sampling devices are also used outside of agriculture. The patent document CN 201218784 Y discloses a device for automatically sampling material during casting processes. The sampling device comprises a crawler chassis on which a sampling mechanism is mounted, comprising a rigid base pivotally connected with two connectors, and the upper ends of which are pivotally connected by a short connector. In the upper part, an arm with a sampling probe is attached to the shorter connector. The arm is moved up and down by means of a mechanical lift with two vertical guides. A rigid base is seated in the vertical guides, which moves up and down together with the connectors connected thereto and the arm with the probe.

Besides, US3789671 discloses a device for sampling particulate material. This device contains a support pole that is attached to a moveable dolly or alternatively may be rigidly connected to a permanent support. The support pole has an arm with a sampling probe. The sampling probe is attached to the arm by a plate which is pivotally connected to said arm. The arm may be rotated about the support pole in order to be position over a bed of said particulate material from which position the probe may be lowered into said material. The support pole is connected to a sleeve by means of a hinge, and said sleeve is connected to the arm with the sampling probe. An actuator in form of a hydraulic piston is pivotally connected to sleeve at the bottom of the sleeve by hinge. Piston rod is pivotally connected at its upper end extending from piston to arm by hinge. As the piston rod is raised the arm is raised and as the piston rod is lowered the arm is lowered.

Document JP1973072838A discloses mechanism of the overload of a crane or the like and an over alarm device. When a hook exceeds a predetermined ascent height in a crane operation, and a load applied to a suspended article is reduced.

What is more, FR2542086A1 discloses a device for taking a samples from a mass of grains. The grain sampling device has a vertical tubular probe, which is connected to a support. Support is connected to a mast by two arms forming an articulated parallelogram. An actuator connects said mast with one of the arms, thus moving probe along a substantially vertical trajectory in order to be raised or lowered. The probe itself is connected by a flexible conduit to a grain receptacle.

A old type of grain sampling probe in known from document US4037476A. In the document the grain sampling probe, comprised of a base, an elongated support pole, a support arm pivotally connected at one end to the top portion of the support pole, an elongated hollow probe pivotally connected to the other end of said support arm, a power output means and a mechanical drive means associated with the power output means to move the probe at a uniform rate of speed downwardly and upwardly so that the probe will obtain a truly representative core sample of the grain or other material within a grain hauling vehicle.

The aim of the invention is to solve the technical problem of the non-linear movement of the probe during the process of driving the probe into the bulk material, which is difficult to precisely control. The aim is also to limit uncontrolled transverse forces acting on the probe during driving into the bulk material.

The invention relates to a mechanism for sampling, especially of bulk materials, having a rigid base connected by at least two rockers to an arm, at the free end of which the probe is pivotally mounted. Rockers are pivotally connected to the base and the arm. The arm is in the form of a rigid beam of constant length, with the first rocker mounted at the end of the arm, and the second rocker attached to the arm. The essence of the invention is in that the length of the boom part of the arm, corresponding to the section between attachment point of the second rocker and the attachment point of the probe is at least 2.5 times greater than the distance between the points of attachment of the first and second rocker to the arm. Furthermore, the quotient of the distance of the point attachment of the probe from the line defined by the points of attachment of the first and second rocker to the arm and the length of the section defined by the points of attachment of the first and second rocker to the base ranges between 0.34 and 0.40.

Preferably:

- the quotient of the length of the second rocker and the length of the section defined by the attachment points of the first and second rocker to the base ranges between 0.21 and 0.29;
- the quotient of the length of the section between the point of attachment of the first rocker to the arm and the point of attachment of the second rocker to the arm and the length of the section defined by the points of attachment of the first and second rocker to the base ranges between 0.58 and 0.73;

- the quotient of the length of the first rocker to the length of the section defined by the attachment point of the first and second rocker to the base ranges between 0.48 and 0.60;
- the quotient of the length of the section between the point of attachment of the second rocker to the arm and the point of attachment of the probe to the arm and the length of the section defined by the points of attachment of the first and second rocker to the base ranges between 1.9 and 2.4;
- the angle between the section defined by the points of attachment of the first and second rocker to the base and the horizontal plane ranges between 32° and 48°.

It is expedient for the mechanism to have a driving mechanism attached thereto.

It is reasonable when the driving mechanism is pivotally mounted on the base and is connected to the arm.

It is especially good when the driving mechanism is attached to the arm behind the attachment point of the second rocker, i.e. the length of the section defined by the point of attachment of the driving mechanism to the arm and the attachment point of the probe to the arm is shorter than the length of the section defined by the point of attachment of the second the rocker to the arm and the attachment point of the probe to the arm.

It is equally good if the base of the mechanism has a bracket on which the driving mechanism is pivotally mounted.

Preferably, the base has a mounting plate.

It is equally appropriate that the arm has a slant arranged between the attachment point of the second rocker and the free end of the arm with the probe so that the free end of the arm with the probe is arranged at an angle relative to the part of the arm that is connected to the first and second rockers..

It is also reasonable for the angle between the part of the arm with the attachment points of both rockers and the part of the arm with the probe to be between 130° and 160°.

The main advantage of the invention is that thanks to the connection of the arm with the base through two rockers and maintaining the size ratios of the individual elements of the mechanism, in the required range of work, during the movement of the arm - and thus the probe - up and down, the probe moves along the path strongly similar in shape to a straight line section. Deviations from straightness may occur, but are not greater than the cross-sectional diameter of the probe. This minimizes the value of the transverse forces acting on the probe during its driving and facilitates the operation of the probe, making the path of the probe's trajectory with high accuracy coincide with the constant vertical axis in the entire working range. Thanks to this design, the up and down movement of the probe and the position of the probe in the bulk material are fully predictable for the operator. This directly translates into the certainty and precision of controlling the probe both when driving it into the bulk material and when operating it over the material and above containers or storage boxes. An additional advantage of the invention is the reduction of the production costs of the device by eliminating the need to use progressive kinematic pairs in the form of telescopic guides enabling linear movement, because the technical implementation of progressive pairs with high transverse load capacity is expensive. Furthermore, the sampling mechanism can cooperate with the driving mechanism, which allows the high functionality of the sampling mechanism to be maintained. Furthermore, the arm with a slant allows for a particularly good adaptation of the sampling mechanism for bulk materials of agricultural origin, where often the probe must be driven into a grain container with high walls. This arm design is particularly useful when taking samples from various types of trailers having sides of different heights.

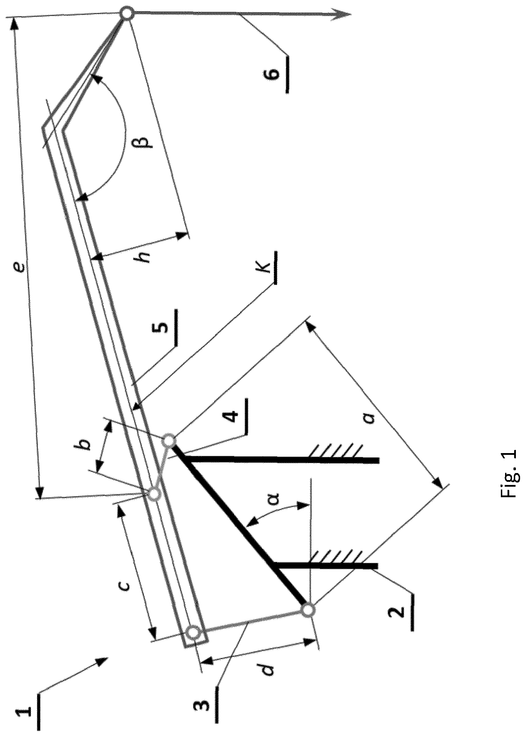

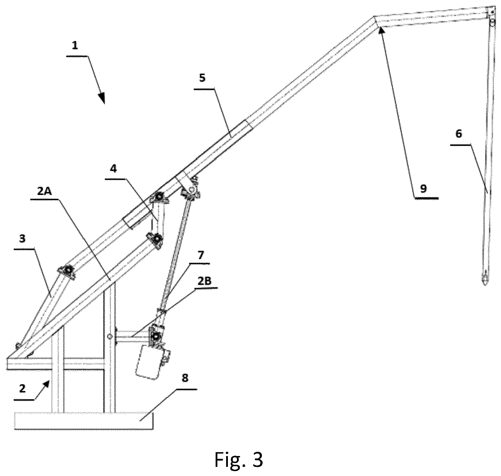

The invention is presented in an embodiment and in the drawing, in which Fig. 1 shows a kinematic diagram of the mechanism, fig. 2 - a kinematic diagram of the mechanism in various positions of the arm, and fig. 3 - the mechanism design in a side view.

A sampling mechanism 1 is designed to collect samples of bulk materials, especially of agricultural origin. It comprises a rigid base 2. The base 2 is formed by a rigid bracket 2A fixedly attached to a horizontal mounting plate 8 by means of posts of different heights. The mounting plate 8 is adapted to be mounted on a mechanical system enabling the plate to be maneuvered in such a way that it is possible to move the sampling mechanism 1 together with the mounting plate 8 along the horizontal axis or to rotate the sampling mechanism 1 together with the mounting plate 8 around the vertical axis.

The rigid bracket 2A of the base 2 is connected by a first rocker 3 and a second rocker 4 to an arm 5 in the form of a rigid beam of constant length. The first and second rockers 3, 4 have a constant length. The first and second rockers 3, 4 are connected to the base 2, i.e. to the rigid bracket 2A, and to the arm 5 in a pivotal manner. The length of the rigid bracket 2A corresponds to the length a of the section defined by the points of attachment of the rockers 3, 4 to the rigid bracket 2A, and thus to the base 2. Additionally, the rigid bracket 2A is attached at an angle $\alpha$ to the mounting plate 8, and thus is inclined relative to the horizontal plane. The angle $\alpha$ between the section defined by the points of attachment of the first and second rockers 3, 4 to the base 2 and the horizontal plane is 40°.

In an embodiment, the first and second rockers 3, 4 and the arm 5 have been implemented as single parts of the mechanism, but in other embodiments it is possible to multiply them, for example doubling the single parts.

In other embodiments, it is possible to make the rockers as double elements mounted on both sides of the arm, which in the kinematic diagrams will be presented as a single functional part, such as a single rocker.

At the free end of the arm 5, a probe 6 is pivotally mounted. The probe 6 mount is a non-driven pivot that enables it to achieve a vertical position due to the force of gravity.

The arm 5 itself, has a slant 9 arranged between the attachment point of the second rocker 4 and the free end of the arm 5 with the probe 6 and two inseparably and immovably connected parts can be distinguished therein. The free end of the arm 5 with the probe 6 is arranged at an angle $\beta$ with respect to the part of the arm 5 that is connected to the first and second rockers 3, 4, and is inclined towards the base 2. In an embodiment, the angle $\beta$ between the part of the arm 5 with the attachment points of the first and second rockers 3, 4 and the part of the arm with the probe 6 is obtuse and equals to 145°. In order to maintain the straightness of the movement of the probe 6, the point of attachment of the probe 6 to the free end of the arm 5 is not collinear with the straight line k determined by the points of attachment of the first rocker 3 and the second rocker 4 to the arm 5. Additionally, the quotient of the distance h of the probe 6 attachment point and line K defined by the points of attachment of the first and second rockers 3, 4 to the arm 5 and the length a of the section defined by the points of attachment of the first and second rockers 3, 4 to the base 2 ranges between 0.34 and 0.40, and in an embodiment it is 0.373. In other embodiments, however, when using a straight arm, to attach the first rocker 3 or the second rocker 4 to the arm, a spacer should be used, moving the attachment point of this connector away from the axis of the arm 5. The second rocker 4 is connected to the arm 5 in such a way in such a way that the length e of the section between the point of attachment of the second connector 4 to the arm 5 and the point of attachment of the probe 6 to the arm 5 is at least 2.5 times greater than the length c of the section between the points of attachment of the first and second rockers 3, 4 to the arm 5. So, the following inequality is valid:

$$\frac{e}{c} > 2.5$$

where:

c - the length of the part of the arm 5 between the points of attachment of the first and second rockers 3, 4 to the arm;

e - the length of the boom part of the arm 5, corresponding to the length of the section between the attachment point of the second rocker 4 and the attachment point of the probe 6.

In an embodiment, the length e of the section between the point of attachment of the second connector 4 to the arm 5 and the point of attachment of the probe 6 to the arm 5 is 3.268 times greater than the length c of the section between the points of attachment of the connectors 3, 4 to the arm 5.

In this description, it is assumed that the length of the section between the attachment point of the second rocker 4 and the attachment point of the probe 6 defines the boom part of the arm 5.

Furthermore, the base 2 includes the bracket 2B of the driving mechanism 7, to which a driving mechanism 7 in the form of an electric linear motor is pivotally connected. On the other side, the electric linear motor is pivotally connected to the arm 5, behind the attachment point of the second rocker 4. This means that the length of the section defined by the point of attachment of the electric linear motor to the arm 5 and by the point of attachment of the probe 6 to the arm 5 is smaller than the length the section defined by the point of attachment of the second rocker 4 to the arm 5 and the point of attachment of the probe 6 to the arm 5.

In other embodiments, any type of actuator may be used as the driving mechanism. The drive can also be directly connected to the plate 8 and also implemented as a rotary drive of the second rocker 4.

In an embodiment, the ratios between the lengths of the individual parts of the sampling mechanism 1 satisfy the following relationships:

- the quotient of the length b of the second rocker 4 and the length of the section defined by the points of attachment of the first and second rockers 3, 4 to the base 2 ranges between 0.21 and 0.29;
- the quotient of the length c of the section between the point of attachment of the first rocker 3 to the arm 5 and the point of attachment of the second connector 4 to the arm 5 and the length a of the section defined by the points of attachment of the first and second rockers 3, 4 to the base 2 ranges between 0.58 a 0.73;

- the quotient of the length d of the first rocker 3 and the length a of the section defined by the points of attachment of the first and second rockers 3, 4 to the base 2 ranges between 0.48 and 0.60;
- the quotient of the length e of the section between the point of attachment of the second rocker 4 to the arm 5 and the point of attachment of the probe to the arm 5 and the length a of the section defined by the points of attachment of the first and second rockers 3, 4 to the base 2 ranges between 1.9 and 2.4;
- the quotient of the distance h of the attachment point of the probe from the line K determined by the points of attachment of the first and second rockers 3, 4 to the arm 5 and the length a of the section defined by the points of attachment of the first and second rockers 3, 4 to the base 2 ranges between 0, 34 a 0.40;
- the angle α between the section defined by the points of attachment of the first and second rockers 3, 4 to the base 2 and the horizontal plane ranges between 32° and 48°;

- the angle β between the part of the arm 5 with the attachment points of the first and second rockers 3, 4 and the part of the arm 5 with the probe 6 ranges between 130° and 160°.

The following conditions have been met in the entire mechanism 1:

$$0.21 < \frac{b}{a} < 0.29$$

$$0.58 < \frac{c}{a} < 0.73$$

$$0.48 < \frac{d}{a} < 0.60$$

$$1.9 < \frac{e}{a} < 2.4$$

$$0.34 < \frac{h}{a} < 0.40$$

$$32° < \alpha < 48°$$

$$130° < \beta < 160°$$

where:

a - the length of the section defined by the points of attachment of the first and second rockers 3, 4 to the base 2;
b - the length of the second rocker 4;
c - the length of the part of the arm 5 between the points of attachment of the first and second rockers 3, 4 to the arm;
d - length of the first rocker 3;
e - the length of the boom part of the arm 5, corresponding to the length of the section between the point of attachment of the second rocker 4 and the point of attachment of the probe 6;
h - the distance of the attachment point of the probe 6 from the line K defined by the points of attachment of the first and second rockers 3, 4 to the arm 5;
α - angle between the section defined by the points of attachment of the first and second rockers 3, 4 to the base 2 and the horizontal plane;
β - angle between the part of the arm 5 with the attachment points of the first and second rockers 3, 4 and the part of the arm 5 with the probe 6.

The above-described ratios between the lengths of the individual parts of the sampling mechanism 1, as well as the above-described angular values, have the following values:

$$\frac{b}{a} = 0.239$$

$$\frac{c}{a} = 0.661$$

$$\frac{d}{a} = 0.536$$

$$\frac{e}{a} = 2.160$$

$$\frac{h}{a} = 0.373$$

$$\alpha = 40°$$

$$\beta = 145°$$

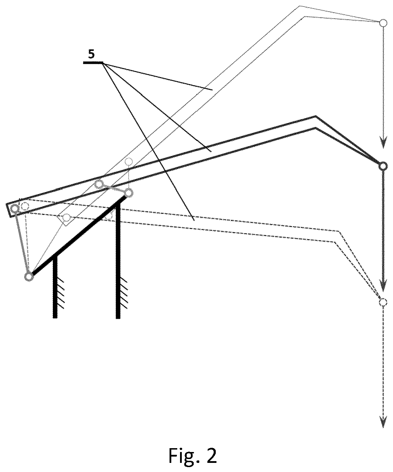

During the operation of the sampling mechanism 1, the driving mechanism 7 moves the arm 5 with the probe 6 up or down, controlling the single kinematic degree of freedom of the sampling mechanism 1. The operation of the driving mechanism 7 and the movement of the arm 5 up or down entails the corresponding swings of the first and second rockers 3, 4. In the entire operating range, the point of pivotal mounting of the probe 6 to the arm 5 moves along a path with a shape very similar to a section of a straight line (Fig. 2). The deviation from straightness is not greater than the cross-sectional diameter of the probe 6. Adoption of such a solution minimizes the size of the transverse forces acting on the probe 6 during its driving and facilitates the operation of the probe 6, making the trajectory of the probe 6 with high accuracy coincide with the constant vertical axis in the entire operating range, and the place of its driving into the bulk material is fully predictable for the operator.

**Claims**

1. A sampling mechanism, especially for bulk materials, having a rigid base (2) connected by at least two rockers (3, 4) to an arm (5), at the free end of which the probe (6) is pivotally connected; wherein the rockers (3, 4) are pivotally connected to a base and the arm, wherein the arm (5) is in the form of a rigid beam of constant length and wherein the first rocker (3) is mounted at the end of the arm (5) and the second rocker (4) is attached to the arm (5), **characterized in that** the length (e) of a boom portion of the arm (5) corresponding to the section between the attachment point of the second rocker (4) and the attachment point of the probe (6) is at least 2.5 times greater than the distance (c) between the points of attachment of the first and second rockers (3, 4) to the arm (5); and, furthermore, the quotient of the distance (h) of the point of attachment of the probe (6) from the line (K) defined by the points of attachment of the first and second rockers (3, 4) to the arm (5) and the length (a) of the section determined by the points of attachment of the first and second rockers (3, 4) to the base (2) ranges between 0.34 and 0.40.

2. The sampling mechanism according to claim 1, wherein

   - the quotient of the length (b) of the second rocker (4) to the length (a) of the section defined by the points of attachment of the first and second rockers (3, 4) to the base (2) ranges between 0.21 and 0.29;
   - the quotient of the length (c) of the section between the point of attachment of the first rocker (3) to the arm (5) and the point of attachment of the second rocker (4) to the arm (5) and the length (a) of the section defined by the points of attachment of the first and second rockers (3, 4) to the base (2) ranges between 0.58 and 0.73;
   - the quotient of the length (d) of the first rocker (3) to the length (a) of the section defined by the points of attachment of the first and second rockers (3, 4) to the base (2) ranges between 0.48 and 0.60;
   - the quotient of the length (e) of the section between the point of attachment of the second rocker (4) to the arm (5) and the point of attachment of the probe (6) to the arm (5) and the length (a) of the section defined by the points of attachment of the first and second rockers (3, 4) to the base (2) ranges between 1.9 and 2.4;
   - the angle ($\alpha$) between the section defined by the points of attachment of the first and second rockers (3), (4) to the base (2) and the horizontal plane ranges between 32° and 48°.

3. The sampling mechanism according to claim 1 or 2, wherein it has a driving mechanism (7) attached thereto.

4. The sampling mechanism according to claim 3, wherein the driving mechanism (7) is pivotally mounted on the base (2) and is connected to the arm (5).

**5.** The sampling mechanism according to claim 4, wherein the driving mechanism (7) is attached to the arm (5) behind the attachment point of the second rocker (4), and the length of the section defined by the point of attachment of the driving mechanism (7) to the arm (5) and the attachment point of the probe (6) to the arm (5) is shorter than the length of the section defined by the attachment point of the second rocker (4) to the arm (5) and the attachment point of the probe (6) to the arm (5).

**6.** The sampling mechanism according to any one of claims from 3 to 5, wherein the base has a bracket (2B) on which the driving mechanism (7) is pivotally mounted.

**7.** The sampling mechanism according to any one of claims from 1 to 6, wherein the base (2) has a mounting plate (8).

**8.** The sampling mechanism according to any one of claims from 1 to 7, wherein the arm (5) has a slant (9) arranged between the attachment point of the second rocker (4) and the free end of the arm (5) with the probe (6) so that the free end of the arm (5) with the probe (6) is arranged at an angle relative to the part of the arm (5) that is connected to the first and second rockers (3, 4).

**9.** The sampling mechanism according to claim 8, wherein the angle ($\beta$) between the part of the arm (5) with the attachment points of the first and second rockers (3, 4) and the part of the arm (5) with the probe (6) ranges between 130° and 160°.

**Patentansprüche**

**1.** Ein Probenahmemechanismus, insbesondere für Schüttgüter, mit einem starren Basis (2), der über mindestens zwei Schwingen (3, 4) mit einem Arm (5) verbunden ist, an dessen freiem Ende die Sonde (6) schwenkbar angebracht ist; wobei die Schwingen (3, 4) schwenkbar mit einem Grundkörper und dem Arm verbunden sind, wobei der Arm (5) die Form eines starren Balkens konstanter Länge hat und wobei die erste Schwinge (3) am Ende des Arms (5) angebracht ist und die zweite Schwinge (4) am Arm (5) befestigt ist, **dadurch gekennzeichnet, dass** die Länge (e) eines Auslegerabschnitts des Arms (5), der dem Abschnitt zwischen dem Befestigungspunkt der zweiten Schwinge (4) und dem Befestigungspunkt der Sonde (6) mindestens 2,5-mal größer ist als der Abstand (c) zwischen den Befestigungspunkten der ersten und zweiten Schwinge (3, 4) am Arm (5); und außerdem der Quotient aus dem Abstand (h) des Befestigungspunkts der Sonde (6) von der Linie (K), die durch die Befestigungspunkte der ersten und zweiten Schwinge (3, 4) am Arm (5) definiert ist, und der Länge (a) des Abschnitts, der durch die Befestigungspunkte der ersten und zweiten Schwinge (3, 4) an dem Basis (2) bestimmt ist, zwischen 0,34 und 0,40 liegt.

**2.** Der Probenahmemechanismus gemäß Anspruch 1, wobei

- der Quotient aus der Länge (b) der zweiten Schwinge (4) und der Länge (a) des Abschnitts, der durch die Befestigungspunkte der ersten und zweiten Schwinge (3, 4) an dem Basis (2) definiert ist, zwischen 0,21 und 0,29 liegt;
- der Quotient aus der Länge (c) des Abschnitts zwischen dem Befestigungspunkt der ersten Schwinge (3) am Arm (5) und dem Befestigungspunkt der zweiten Schwinge (4) am Arm (5) und der Länge (a) des Abschnitts, der durch die Befestigungspunkte der ersten und zweiten Schwinge (3, 4) an dem Basis (2) definiert ist, zwischen 0,58 und 0,73 liegt;
- der Quotient aus der Länge (d) der ersten Schwinge (3) und der Länge (a) des Abschnitts, der durch die Befestigungspunkte der ersten und zweiten Schwinge (3, 4) an dem Basis (2) definiert ist, zwischen 0,48 und 0,60 liegt;
- der Quotient aus der Länge (e) des Abschnitts zwischen dem Befestigungspunkt der zweiten Schwinge (4) am Arm (5) und dem Befestigungspunkt der Sonde (6) am Arm (5) und der Länge (a) des Abschnitts, der durch die Befestigungspunkte der ersten und zweiten Schwinge (3, 4) an dem Basis (2) definiert ist, zwischen 1,9 und 2,4 liegt;
- der Winkel ($\alpha$) zwischen dem Abschnitt, der durch die Befestigungspunkte der ersten und zweiten Schwinge (3), (4) an dem Basis (2) definiert ist, und der horizontalen Ebene liegt zwischen 32° und 48°.

**3.** Der Probenahmemechanismus nach Anspruch 1 oder 2, wobei daran ein Antriebsmechanismus (7) angebracht ist.

**4.** Der Probenahmemechanismus nach Anspruch 3, wobei der Antriebsmechanismus (7) schwenkbar an dem Basis (2) angebracht und mit dem Arm (5) verbunden ist.

**5.** Der Probenahmemechanismus nach Anspruch 4, wobei der Antriebsmechanismus (7) an dem Arm (5) hinter dem Befestigungspunkt der zweiten Schwinge (4) angebracht ist und die Länge des Abschnitts, der durch den Befestigungspunkt des Antriebsmechanismus (7) an dem Arm (5) und dem Befestigungspunkt der Sonde (6) an dem Arm (5) kürzer ist als die Länge des Abschnitts, der durch den Befestigungspunkt der zweiten Schwinge (4) an dem Arm (5) und den Befestigungspunkt der Sonde (6) an dem Arm (5) definiert ist.

6. Der Probenahmemechanismus gemäß einem der Ansprüche 3 bis 5, wobei der Grundkörper eine Halterung (2B) aufweist, an der der Antriebsmechanismus (7) schwenkbar angebracht ist.

7. Der Probenahmemechanismus nach einem der Ansprüche 1 bis 6, wobei der Basis (2) eine Montageplatte (8) aufweist.

8. Der Probenahmemechanismus nach einem der Ansprüche 1 bis 7, wobei der Arm (5) eine Schräge (9) aufweist, die zwischen dem Befestigungspunkt der zweiten Schwinge (4) und dem freien Ende des Arms (5) mit der Sonde (6) so angeordnet ist, dass das freie Ende des Arms (5) mit der Sonde (6) in einem Winkel relativ zu dem Teil des Arms (5) angeordnet ist, der mit der ersten und zweiten Schwinge (3, 4) verbunden ist.

9. Der Probenahmemechanismus nach Anspruch 8, wobei der Winkel (β) zwischen dem Teil des Arms (5) mit den Befestigungspunkten der ersten und zweiten Schwinge (3, 4) und dem Teil des Arms (5) mit der Sonde (6) zwischen 130° und 160° liegt.

**Revendications**

1. Un mécanisme d'échantillonnage, en particulier pour les matériaux en vrac, comportant une base rigide (2) reliée par au moins deux bascules (3, 4) à un bras (5), à l'extrémité libre duquel la sonde (6) est reliée de manière pivotante ; dans lequel les bascules (3, 4) sont reliées de manière pivotante à une base et au bras, dans lequel le bras (5) se présente sous la forme d'une poutre rigide de longueur constante et dans lequel la première bascule (3) est montée à l'extrémité du bras (5) et la seconde bascule (4) est attachée au bras (5),**caractérisé en ce que** la longueur (e) d'une partie de flèche du bras (5) correspondant à la section entre le point d'attache de la seconde bascule (4) et le point d'attache de la sonde (6) est au moins 2,5 fois plus grande que la distance (c) entre les points d'attache de la première et de la seconde bascule (3, 4) au bras (5) ; et, en outre, le quotient de la distance (h) du point d'attache de la sonde (6) par rapport à partir de la ligne (K) définie par les points d'attache de la première et de la seconde bascule (3, 4) au bras (5) et de la longueur (a) de la section déterminée par les points d'attache de la première et de la seconde bascule (3, 4) à la base (2) est compris entre 0,34 et 0,40.

2. Le mécanisme d'échantillonnage selon la revendication 1, dans lequel

> - le quotient de la longueur (b) de la seconde bascule (4) par la longueur (a) de la section définie par les points d'attache de la première et de la seconde bascule (3, 4) à la base (2) est compris entre 0,21 et 0,29 ;
> - le quotient de la longueur (c) de la section entre le point d'attache de la première bascule (3) au bras (5) et le point d'attache de la seconde bascule (4) au bras (5) et la longueur (a) de la section définie par les points d'attache de la première et de la seconde bascule (3, 4) à la base (2) est compris entre 0,58 et 0,73 ;
> - le quotient de la longueur (d) de la première bascule (3) par la longueur (a) de la section définie par les points d'attache de la première et de la seconde bascule (3, 4) à la base (2) est compris entre 0,48 et 0,60 ;
> - le quotient de la longueur (e) de la section entre le point d'attache de la seconde bascule (3) au bras (5) et le point d'attache de la sonde (6) au bras (5) et la longueur (a) de la section définie par les points d'attache de la première et de la seconde bascule (3, 4) à la base (2) est compris entre 1,9 et 2,4 ;
> - l'angle (α) entre la section définie par les points d'attache de la première et de la seconde bascule (3), (4) à la base (2) et le plan horizontal est compris entre 32° et 48°.

3. Le mécanisme d'échantillonnage selon la revendication 1 ou 2, dans lequel il a un mécanisme d'entraînement (7) attaché à celui-ci.

4. Le mécanisme d'échantillonnage selon la revendication 3, dans lequel le mécanisme d'entraînement (7) est monté de manière pivotante sur la base (2) et est relié au bras (5).

5. Le mécanisme d'échantillonnage selon la revendication 4, dans lequel le mécanisme d'entraînement (7) est attaché au bras (5) derrière le point d'attache de la seconde bascule (4), et la longueur de la section définie par le point d'attache du mécanisme d'entraînement (7) au bras (5) et le point d'attache de la sonde (6) au bras (5) est plus courte que la longueur de la section définie par le point d'attache de la seconde bascule (4) au bras (5) et le point d'attache de la sonde (6) au bras (5).

6. Le mécanisme d'échantillonnage selon l'une des revendications 3 à 5, dans lequel la base a un support (2B) sur lequel le mécanisme d'entraînement (7) est monté de manière pivotante.

7. Le mécanisme d'échantillonnage selon l'une des revendications 1 à 6, dans lequel la base (2) a une

**EP 4 300 068 B1**

plaque de montage (8).

8. Le mécanisme d'échantillonnage selon l'une des revendications 1 à 7, dans lequel le bras (5) a une inclinaison (9) entre le point d'attache de la seconde bascule (4) et l'extrémité libre du bras (5) avec la sonde (6) de sorte que l'extrémité libre du bras (5) avec la sonde (6) est disposée à un angle par rapport à la partie du bras (5) qui est reliée aux première et seconde bascules (3, 4).

9. Le mécanisme d'échantillonnage selon la revendication 8, dans lequel l'angle (β) entre la partie du bras (5) avec les points d'attache des première et seconde bascules (3, 4) et la partie du bras (5) avec la sonde (6) est compris entre 130° et 160°.

Fig. 1

Fig. 2

Fig. 3

**EP 4 300 068 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 285485 U
- DE 3612410 A1
- CN 201218784 Y
- US 3789671 A
- JP 1973072838 A
- FR 2542086 A1
- US 4037476 A